# EUROPÄISCHE PATENTANMELDUNG

(11) **EP 1 491 635 A2**
(43) Veröffentlichungstag der Anmeldung: **29.12.2004**
(21) Anmeldenummer: 04003837.4
(22) Anmeldetag: 20.02.2004
(51) Int. Cl.: C12Q 1/00

(54) **Reahenzloser Biosensor zum Nachweis von Nitrilen und Cyaniden**

(30) Priorität: 20.03.2003 DE 10312296
(71) Anmelder: FORSCHUNGSZENTRUM JÜLICH GMBH, 52425 Jülich (DE)
(72) Erfinder: Schöning, Michael, Josef, 52428 Jülich (DE); Keusgen, Michael, 53359 Rheinbach (DE); Jünger, Martina, 53619 Rheinbreitbach (DE)

(57) **Zusammenfassung**

Die Erfindung betrifft einen Biosensor (1) zum Nachweis von Nitrilen und Cyaniden.

Der Biosensor (1) umfasst einen dotierten Halbleiter (3), mindestens einen auf dem Halbleiter (3) aufgebrachten pH-sensitiven Isolator (5) und ein auf dem Isolator (5) immobilisiertes Enzym. Das Enzym ist eine Nitrilase, insbesondere eine Cyanidase (6) aus *Pseudomonas stuzeri*.

Bei Testmessungen konnte eine Empfindlichkeit von besser als 1x10⁻⁶ M (26 µg CN⁻ je Liter) erzielt werden.

## Beschreibung

Die Erfindung betrifft einen Biosensor zum Nachweis von Nitrilen und Cyaniden.

Als Nitrile werden die Alkylderivate der Blausäure (HCN) aufgefaßt.
Als Cyanide werden die Salze der Blausäure aufgefaßt.

Aus dem Stand der Technik sind verschiedene Methoden zum Nachweis von Cyaniden bekannt.

Cyanid-Sensoren auf der Basis von ionensensitiven Elektroden (Cyanid-ISE) werden kommerziell von verschiedenen Herstellern angeboten. Die Sensoren umfassen eine sensitive Schicht aus einer Silberhalogenidverbindung zusammen mit einer Ag₂S-Verbindung.
Nachteilig sind diese Sensoren nur in einem pH-Bereich von 9-11 einsetzbar und zeigen konstruktionsbedingt starke Interferenzen mit Sulfid (S²⁻), Halogeniden (Cl⁻, Br⁻, F⁻, etc.) und Silber (Ag⁺). Aufgrund von Komplexbildungsprozessen verbraucht sich die Elektrode während der Messzeit und ist bereits nach wenigen Einsätzen nicht mehr zuverlässig benutzbar. Eine Miniaturisierung solcher ISEs ist bisher nur in begrenztem Umfang möglich.

Aus dem Stand der Technik sind weiterhin Cyanid-Biosensoren bekannt.

Es ist bekannt, daß Enzyme, die Eisen oder Kupfer als Zentralatom enthalten, durch Cyanide stark gehemmt werden. Cyanid-Sensoren auf Basis enzymatischer Nachweisreaktionen weisen die in einer Lösung befindlichen Substrate nach enzymatischer Umsetzung nach.
Nachteilig sind derartige Sensoren unspezifisch. Es werden alle Enzyminhibitoren in der Testlösung in gleicher Weise erfaßt.
Weiterhin nachteilig lässt sich ein solcher Sensor nur mit hohem Aufwand für Realzeit-Messungen im Feld einsetzen. Hierzu muß der Biosensor zunächst mit seiner substrateigenen Lösung, korrespondierend zum Enzym, auf einen konstanten Wert eingestellt werden (konstante Umsatzrate, z.B. ΔpH). Danach erfolgt die Inhibierung durch das Substrat Cyanid durch messbare Veränderung des pH. Danach muss der Sensor wieder reaktiviert werden, z.B. in einer hohen Substratkonzentration.

Aus Lee et al. ist ein Sensor auf Basis ganzer Zellen bekannt (Lee, J.I., Karube, I. (1995) A novel microbial sensor for the determination of cyanide. Analytica Chimica Acta 313: 69-74).
Cyanide bringen den Zellstoffwechsel durch Inhibierung der Enzyme zum Erliegen, was zu einem verminderten Sauerstoffverbrauch der Zelle führt. Andererseits kommt es bei Zellen, die über das Enzym Cyanidase verfügen, zu einer gesteigerten Sauerstoff-Aufnahme, die mit einer Sauerstoff-Elektrode verfolgt werden kann.
Diese Methode reagiert jedoch auf alle weiteren Stoffe, die den Zellstoffwechsel beeinflussen. Die Handhabung der Zellen erfordert einen hohen experimentellen Aufwand, um einen solchen Biosensor in der Praxis zu betreiben. Außerdem muss die sensoraktive Schicht mit einer Sauerstoffelektrode gekoppelt werden, was einer einfachen und miniaturisierten Herstellung des Biosensors im Wege steht.

Weiterhin sind verschiedene Biosensoren mit immobilisierten Enzymen aus dem Stand der Technik bekannt.

Aus DE 44 36 001 C2 ist ein Biosensor mit auf einer Si₃N₄-Oberfläche fixiertem Enzym bekannt, bei der das Enzym kovalent an diese Oberfläche mit Hilfe eines heterobifunktionalen Crosslinkers angebunden ist. Nachteilig lässt sich ein derartiger Biosensor nur durch Verwendung einer teueren oberflächenchemischen Behandlung herstellen. Es lassen sich durch das angegebene Verfahren auch nur Monoschichten des Enzyms herstellen. Das Herstellungsverfahren ist bei Verwendung von Enzymen, die eine niedrige Aktivität aufweisen, ungeeignet.

Aus Keusgen et al. (Michael Keusgen, Peter Milka und Ingo Krest, 2001. Entwicklung eines Cyanid-Biosensors: Cyanidase aus bakteriellen Quellen. Zweites Biosensorsymposium Tübingen, 2001; http://barolo.ipc.unituebingen.de/biosensor2001) ist eine Durchfluss-Messapparatur zum Nachweis von Cyanid (Blausäure) auf der Basis von immobilisierter Cyanidase mittels einer Ammoniak-Elektrode bekannt. Hierbei sind die Elektrode und das Enzym nicht direkt miteinander verbunden. Cyanidasen gehören zu der Gruppe der Nitrilasen, EC 3.5.5.1. Während des Nachweisverfahrens wird Cyanid in einer Probe enzymatisch zu Ammoniak und Ameisensäure umgesetzt. Ammoniak wird anschließend mit einer Ammoniak-Elektrode erfasst.
Auch bei diesem Sensor ist eine Miniaturisierung wegen der Ammoniak-Messelektrode nicht möglich.

Aufgabe der Erfindung ist es einen miniaturisierten Biosensor zum sensitiven Nachweis von Nitrilen, und insbesondere Cyaniden bereit zu stellen.

Die Aufgabe wird durch einen Biosensor gemäß Hauptanspruch gelöst. Vorteilhafte Ausgestaltungen ergeben sich aus den darauf rückbezogenen Patentansprüchen.
Der Biosensor umfasst eine dotierte Halbleiterschicht, insbesondere aus p- oder n-dotiertem Silizium, mindestens einem auf der Halbleiterschicht aufgebrachten pH-sensitiven Isolator und einer auf dem Isolator immobilisierten Nitrilase aus der Substanzklasse EC 3.5.5.1. Die Enzymklasse der Nitrilasen umfasst Cyanidasen. Als eine Nitrilase kann also auch eine Cyanidase immobilisiert sein.
Ein derartiger Biosensor detektiert Nitrile, also die Alkylderivate der Blausäure, z. B. Mandelonitril, und Cyanide, wie Blausäure und deren Salze ohne Hilfsreagenzien. Das heißt, es wird erstmalig ein echter Biosensor zum Nachweis dieser Substanzen vorgestellt. Der Sensor ist vorteilhaft auf Grund der Halbleiterschichtstruktur miniaturisiert. Querempfindlichkeiten zu anderen Ionen, wie z. B. bei der Cyanid-ISE, sind ausgeschlossen.

Als Nitrilasen eignen sich prinzipiell alle Enzyme der Enzymklasse EC 3.5.5.1. Diese Enzyme unterscheiden sich in ihrer Substratspezifität. Als Nitrilase kann also auch eine Cyanidase, die spezifisch Cyanide, also die Salze der Blausäure, umsetzt, immobilisiert sein. Demgemäß können auch Cyanide wie Blausäure hydrolisiert und quantitativ nachgewiesen werden. Die Reaktion hängt maßgeblich von der Substratspezifität des verwendeten Enzyms ab. Geeignete Enzyme können aus bakteriellen, pilzlichen, pflanzlichen und tierischen Quellen gewonnen werden.

Vorteile gegenüber Sensoren gemäß Stand der Technik sind die hohe Empfindlichkeit und die Miniaturisierung. Es werden keine Hilfsreagenzien benötigt, das heißt der Sensor kann auch in die Untersuchungslösung eingetaucht werden. Man ist also mit einem derartigen Biosensor sehr flexibel bezüglich der Messung. Es werden zudem nur geringe Enzymmengen benötigt. Der Sensor ist zum oftmaligen Gebrauch geeignet und wird vorteilhaft in einem pH-Bereich zwischen 7,0 und 8,5 betrieben.

Eine bestimmte Cyanidase stammt besonders vorteilhaft aus *Pseudomonas stuzeri* (AK 61).
Diese Cyanidase ist stabil auf einem pH-sensitiven Isolator immobilisierbar. Diese Cyanidase weist vorteilhaft auch die Eigenschaft auf, mit verschiedenartigen Verfahren an den pH-sensitiven Isolator immobilisierbar zu sein.

Der Biosensor kann Mittel aufweisen, die die Nitrilase mechanisch an dem pH-sensitiven Isolator immobilisieren. Als Mittel ist z. B. eine Dialysemembran mit einer Ausschlußgröße von 10 kDa besonders geeignet. In diesem Fall passieren niedermolekulare Analyte (Cyanid, Nitrile) die Membran ungehindert. Das Enzym kann hingegen nicht in die Untersuchungslösung entweichen. Die Dialysemembran kann z. B. über einen O-Ring gegen den pH-sensitiven Isolator abgedichtet sein.

Das Enzym kann vor der Immobilisierung auf dem pH-sensitiven Isolator an ein Gel oder an einen Feststoff chemisch z. B. durch kovalente Bindung oder durch absorptive Bindung verbunden werden. Dann kann das Gel oder der Feststoff samt Enzym z. B. mittels mechanisch immobilisierender Mittel an den pH-sensitiven Isolator immobilisiert werden.

In einer weiteren Ausgestaltung der Erfindung kann die Nitrilase vor der Immobilisierung an ein Gel oder einen Feststoff fixiert sein, das einen Polyzucker umfaßt. Hierfür sind insbesondere Polyzucker wie Agarose, Cellulose, Dextran und deren Derivate geeignet.

Die Nitrilase kann aber auch auf dem pH-sensitiven Isolator in einer Lösung gelöst vorliegen und mittels mechanischer Mittel, wie einer Dialysemembran, an den pH-sensitiven Isolator immobilisiert sein.

Grundsätzlich läßt sich die Nitrilase aber auch chemisch über Amino-, Hydroxy-, Thio- oder Carboxyfunktionen der Cyanidase direkt an den pH-sensitiven Isolator immobilisieren. Die Nitrilase kann zudem auch absorptiv an den pH-sensitiven Isolator immobilisiert sein.

Es ist somit möglich, verschiedene Immobilisierungsverfahren miteinander zu koppeln, also beispielsweise die Nitrilase zunächst chemisch an ein Makromolekül, z. B. einen Polyzucker, zu binden und sodann diese Verbindung z. B. mit einer Dialysemembran auf dem pH-sensitiven Isolator mechanisch zu immobilisieren.

Zwischen dem pH-sensitiven Isolator und der Halbleiterschicht kann eine weitere Isolatorschicht insbesondere aus SiO₂ angeordnet sein.

In einer weiteren Ausgestaltung der Erfindung umfaßt der Biosensor Si₃N₄ als Material für den pH-sensitiven Isolator. Es lassen sich aber genauso gut andere pHsensitive Dünnschichtmaterialien wie Ta₂O₅, Al₂O₃ oder ZrO₂ oder SiO₂ selbst einsetzen.

Der Biosensor kann sowohl als geschlossene Meßkammer als auch als Durchfluss-Messkammer ausgestaltet sein.
Der Biosensor ist auf der Basis eines Feldeffektsensors ausgebildet. Der Biosensor ist insbesondere als kapazitive Elektrolyt-Isolator-Halbleiter-Struktur (EIS-Sensor) ausgebildet. Das heißt, zur Messung des Analyten taucht eine Referenzelektrode in die zu untersuchende Lösung ein. Diese steht in Kontakt mit dem immobilisierten Enzym. Zur vollständigen pH- und Temperaturkompensation ist nur ein weiterer EIS-Sensor ohne Enzymbeschichtung erforderlich.
Ein EIS-Sensor weist eine Enzymschicht, hier die Cyanidase bzw. Nitrilase auf und mißt als Endprodukt der enzymatischen Reaktion die Veränderung des pH-Wertes. Ein weiterer EIS-Sensor weist eine entsprechende Struktur ohne Enzym auf. Dieser weitere EIS-Sensor mißt keine Änderungen des pH-Wertes durch die enzymatische Reaktion aber eventuell auftretende Änderungen des pH-Wertes in der Meßlösung. Dadurch ist eine Kalibration des Sensors möglich. Durch Differenzbildung wird das durch die enzymatische Reaktion hervorgerufene Signal erfaßt. Auf dieselbe Art und Weise läßt sich auch die Temperaturabhängigkeit eliminieren.

An Stelle eines kapazitiven EIS-Sensors kann der Sensor aber auch als Feldeffektstruktur, das heißt als ISFET (ionensensitiver Feldeffekttransistor) oder auch als LAPS (lichtadressierbarer potentiometrischer Sensor) ausgelegt sein. Ein Fachmann kann durch einfache Versuche einen jeweils geeigneten Sensoraufbau konstruieren.

Im weiteren wird die Erfindung an Hand eines Ausführungsbeispiels und der beigefügten zwei Figuren näher beschrieben.

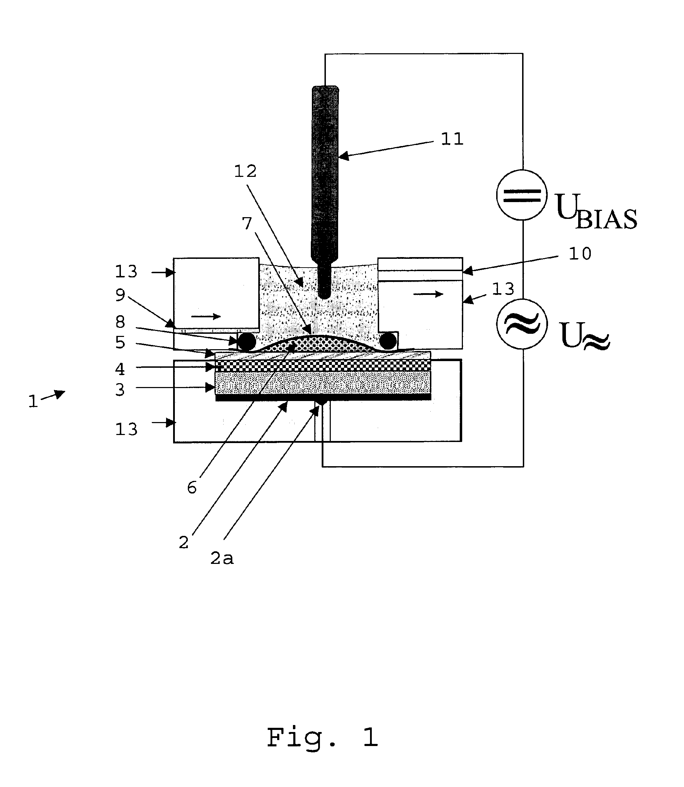

Figur 1 zeigt schematisch den Aufbau eines Biosensors 1 zum Nachweis von Cyanid (Blausäure). Der Sensor 1 ist als EIS-Struktur ausgebildet. Er umfaßt einen dotierten Halbleiter 3, z. B. p- oder n-dotiertes Silizium, der an der Unterseite eine Metallschicht 2 aus z. B. Aluminium aufweist und über einen externen Kontakt 2a aus z. B. Gold mit einer Messeinrichtung verbunden ist. Auf der Oberseite des p-Siliziums 3 ist eine Schicht aus Siliziumdioxid (SiO₂) 4, auf der wiederum eine pHsensitive Schicht aus Siliziumnitrit (Si₃N₄) 5 angeordnet ist. Auf dieser pH-sensitiven Schicht 5 ist eine Cyanidase 6 aus *Pseudomonas stuzeri* (AK 61) mit einer Dialysemembran 7 einer Ausschlussgröße von 10 kDa mechanisch immobilisiert. Die Abdichtung erfolgt über einen O-Ring 8. Die Cyanidase 6 ist kovalent an ein Gel oder einen Feststoff fixiert. Ein Agarose-Derivat als Polyzucker ist hierzu besonders geeignet. Die Fixierung an das Agarose-Derivat erfolgte somit auf chemischem Wege über die Aminofunktionen des Enzyms 6 an einen funktionalisierten Polyzucker über eine Säureamid-Bindung. Die funktionalisierten Gruppen, z. B. Carboxy-Funktionen, ermöglichen die kovalente Bindung.
Die Immobilisierung auf der Si₃N₄-Schicht 5 erfolgte mechanisch mittels Dialysemembran 7 und O-Ring 8.

Im vorliegenden Fall ist der Biosensor 1 in eine Durchfluss-Messkammer 13 aus einem nichtleitenden Polymerwerkstoff eingebaut. Ein Zufluss 9 und ein Abfluss 10 stellen den Eintritt und Austritt einer zu untersuchenden Lösung mit dem Analyten dar. Der Biosensor 1 kann aber auch in einer geschlossenen Messkammer betrieben werden. Die für die Messungen erforderliche Referenzelektrode 11, z. B. eine Silber/Silberchlorid-Elektrode, kann aber auch einfach nur in eine Untersuchungslösung 12 eingetaucht werden. Man ist also mit einem derartigen Biosensor 1 bezüglich der Messung sehr flexibel.

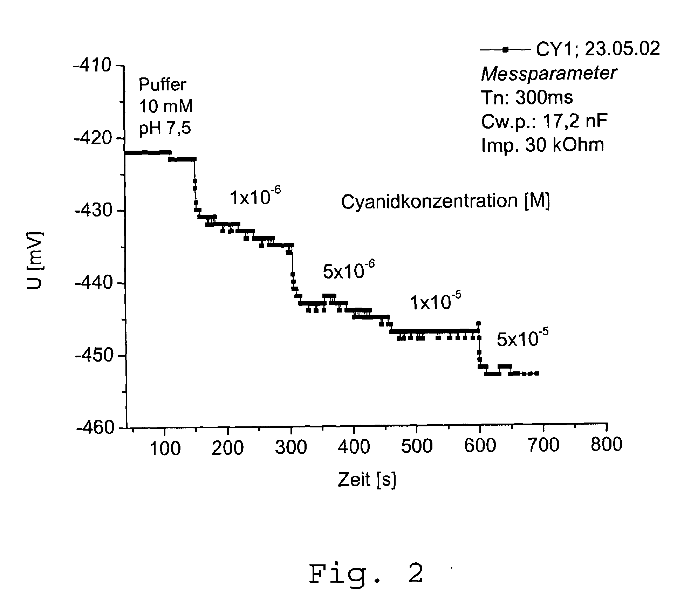

Fig. 2 zeigt exemplarisch ein Sensogramm des EIS-Biosensors bei unterschiedlichen Cyanid Konzentrationen (Sensorbezeichnung: CY1, Kapazität 17,2 nF, Impedanz 30 kOhm, Zeitkonstante für die Messungen 300 ms). Die Messung wurde im sogenannten CONCAP-Modus durchgeführt, das heißt bei konstanter Kapazität der EIS-Struktur (17,2 nF). Bei den Messungen wurde das Potential (U, mV) gegen die Zeit (s) aufgetragen. Es wurde die in Fig. 1 dargestellte Durchfluss-Messzelle verwendet, wobei mit einer Durchflussrate von 0,25 ml/min bei einem pH-Wert von 7.5 (Phosphatpuffer, Molarität 10 mM) gearbeitet wurde. Für die Versuche wurde die Cyanidase des Bakteriums *Pseudomonas stuzeri* verwendet (6 Units), die an 20 mg eines Agarose-Derivates kovalent gebunden war. Die Erfassung des Messsignals erfolgt hier mittels C/V-(Kapazitäts/Spannungs-)Messung oder Concap-Messung bei konstanter Kapazität.

Es lassen sich aber nach Anpassung der Ansteuerungselektronik auch andere für Feldeffektsensoren bekannten Messverfahren, wie z. B. Photostrommessungen oder Strom/Spannungsmessungen durchführen.

Es konnte eine Empfindlichkeit von besser als 1x10⁻⁶ M (26 µg CN⁻ je Liter) nachgewiesen werden.

## Patentansprüche

1. Biosensor (1) zum Nachweis von Nitrilen und / oder Cyaniden,
umfassend einen dotierten Halbleiter (3), mindestens einem auf dem Halbleiter (3) aufgebrachten pH-sensitiven Isolator (5) und einem auf dem Isolator (5) immobilisierten Enzym,
**gekennzeichnet durch**
eine Nitrilase aus der Substanzklasse EC 3.5.5.1 als Enzym.

2. Biosensor (1) nach Anspruch 1,
**gekennzeichnet durch**
eine Cyanidase (6) als Nitrilase.

3. Biosensor (1) nach einem der vorhergehenden Ansprüche,
**gekennzeichnet durch**
eine Cyanidase (6) aus *Pseudomonas stuzeri* (AK 61) als Nitrilase.

4. Biosensor (1) nach einem der vorhergehenden Ansprüche,
**dadurch gekennzeichnet, dass**
der Sensor Mittel (7, 8) aufweist, die die Nitrilase mechanisch auf dem pH-sensitiven Isolator (5) immobilisieren.

5. Biosensor (1) nach einem der vorhergehenden Ansprüche,
**gekennzeichnet durch**
eine Dialysemembran (7) und einen O-Ring (8) als Mittel, die die Nitrilase auf dem pH-sensitiven Isolator (5) mechanisch immobilisieren.

6. Biosensor (1) nach einem der vorhergehenden Ansprüche,
**dadurch gekennzeichnet, dass**
die Nitrilase an ein Gel oder einen Feststoff kovalent oder absorptiv gebunden ist.

7. Biosensor (1) nach einem der vorhergehenden Ansprüche,
**dadurch gekennzeichnet, dass**
die Nitrilase an einen Polyzucker oder an ein Derivat eines Polyzuckers kovalent gebunden ist.

8. Biosensor (1) nach einem der vorhergehenden Ansprüche,
**dadurch gekennzeichnet, dass**
die Nitrilase in einer Lösung auf dem pH-sensitiven Isolator (5) vorliegt.

9. Biosensor (1) nach einem der vorhergehenden Ansprüche,
**dadurch gekennzeichnet, dass**
die Nitrilase chemisch an dem pH-sensitiven Isolator (5) immobilisiert ist.

10. Biosensor (1) nach einem der vorhergehenden Ansprüche,
**dadurch gekennzeichnet, dass**
die Nitrilase absorptiv an den pH-sensitiven Isolator (5) gebunden ist.

11. Biosensor (1), nach einem der vorhergehenden Ansprüche,
**gekennzeichnet durch**
einen Halbleiter (3) aus p- oder n-dotiertem Silizium.

12. Biosensor (1) nach einem der vorhergehenden Ansprüche,
**dadurch gekennzeichnet, dass**
zwischen dotierten Halbleiter (3) und pH-sensitiven Isolator (5) eine SiO₂-Schicht (4) angeordnet ist.

13. Biosensor (1) nach einem der vorhergehenden Ansprüche,
**gekennzeichnet durch**
eine Metallschicht (2) und einen externen Kontakt (2a) an der Unterseite des dotierten Halbleiters (3).

14. Biosensor (1) nach einem der vorherigen Ansprüchen,
**dadurch gekennzeichnet, dass**
eine Referenzelektrode (11) in eine auf Nitrile oder Cyanide zu analysierende Lösung (12) eintaucht.

15. Biosensor (1) nach einem der vorhergehenden Ansprüche,
**gekennzeichnet durch**
Si₃N₄ oder Ta₂O₅ oder Al₂O₃ oder ZrO₂ oder SiO₂ als Material für den pH-sensitiven Isolator (5).

16. Biosensor (1) nach einem der vorhergehenden Ansprüche,
**dadurch gekennzeichnet, dass**
dieser als geschlossene Meßkammer ausgestaltet ist.

17. Biosensor (1) nach einem der vorhergehenden Ansprüche 1 bis 15,
**dadurch gekennzeichnet, dass**
der Sensor als Durchfluss-Messkammer (13) mit Zulauf (9) und Ablauf (10) ausgestaltet ist.

18. Biosensor (1) nach einem der vorhergehenden Ansprüche,
**gekennzeichnet durch**
Mittel zur pH- und Temperaturkompensation.

19. Biosensor (1) nach einem der vorhergehenden Ansprüche 1 bis 18,
**dadurch gekennzeichnet, dass**
dieser als ionensensitiver Feldeffekttransistor ausgebildet ist.

20. Biosensor (1) nach einem der vorhergehenden Ansprüche,
**dadurch gekennzeichnet, dass**
dieser als EIS-Struktur ausgebildet ist.

21. Biosensor (1) nach einem der vorhergehenden Ansprüche,
**dadurch gekennzeichnet, dass**
dieser als lichtadressierbarer potentiometrischer Sensor ausgebildet ist.
